# EUROPÄISCHE PATENTSCHRIFT

(11) **EP 3 231 393 B1**
(45) Veröffentlichungstag und Bekanntmachung des Hinweises auf die Patenterteilung: **21.06.2023**
(21) Anmeldenummer: 16165141.9
(22) Anmeldetag: 13.04.2016
(51) Int. Cl.: A61F 2/24

(54) **MINIMAL-INVASIV IMPLANTIERBARE MITRAL- UND TRIKUSPIDALKLAPPE**
MINIMALLY INVASIVE IMPLANTABLE MITRAL AND TRICUSPID VALVE
VALVE TRICUSPIDE ET MITRALE IMPLANTABLE A INVASION MINIMALE

(43) Veröffentlichungstag der Anmeldung: 18.10.2017
(73) Patentinhaber: Vallbracht, Christian, 36251 Bad Hersfeld (DE)
(72) Erfinder: Vallbracht, Christian, 36251 Bad Hersfeld (DE)
(74) Vertreter: Zellentin & Partner mbB Patentanwälte

(56) Entgegenhaltungen:
- EP-A1- 3 000 437
- US-A1- 2006 271 172
- US-A1- 2007 066 993
- US-A1- 2007 156 233
- US-A1- 2014 031 928
- US-A1- 2014 330 367

## Beschreibung

Die vorliegende Erfindung betrifft eine Mitralklappe oder Trikuspidalklappe, welche sich mittels minimal-invasiver Technik implantieren lässt. Insbesondere handelt es sich um eine perkutan implantierbare selbstexpandierende Mitral- oder Trikuspidalbioklappe an einem selbstexpandierenden Vorhofstent. Optional erfolgt ein Verschluss des Vorhofohres und/oder eines Vorhofseptumdefektes mit Patches, z.B. PTFE-Patches.

Die natürlichen Herzklappen, Aortenklappe, Mitralklappe, Pulmonalklappe und Trikuspidalklappe, sind für die Pumpfunktion des Herzens von zentraler Bedeutung, indem sie den Blutfluss kontrollieren. Vielfältige Erkrankungen können zu verschiedenen Schädigungen führen, welche die Funktion der Klappen beeinträchtigen oder gar zum Erliegen kommen lassen. In solchen Fällen kann nur eine Reparatur oder der Ersatz der defekten Klappe helfen. Künstliche Herzklappen sind seit langem bekannt und zur Behandlung von Erkrankungen der Herzklappen im Einsatz. Zunächst war eine Operation am offenen Herzen für diese Eingriffe nötig, die für viele Pateinten wegen der damit verbundenen Belastung des ohnehin schon schwachen Herzens nicht durchführbar war.

Dieses Problem wurde mit der Entwicklung minimal-invasiver Techniken gelöst, bei denen der Eingriff perkutan, z.B. transfemoral durch die Leistenarterie oder transapikal nach Inzision durch die Herzspitze, erfolgt. So sind seit einigen Jahren perkutan implantierbare, ballondehnbare oder selbstexpandierende Bioklappen für die Behandlung der zumeist verkalkten Aortenklappenstenose verfügbar, siehe z.B. die Erstbeschreibung durch Alain Cribier et al., "Percutaneous transcatheter implantation of an aortic valve prothesis for calcific aortic stenosis: first human case description", Circulation 106 (24), 2002, S.3006-8.

Für die zweithäufigste Klappenerkrankung beim Erwachsenen, die Mitralklappeninsuffizienz, sind mehrere unterschiedliche Modelle in experimenteller Erprobung. Darüberhinaus sind viele verschiedene Vorschläge gemacht worden, beispielsweise US 2010/217382 A1, WO 2010/101190 A1, US 2004/0138745 A1, EP 2 982 336 A1, WO 2011/137531 A1, WO 2011/044994 A1, DE 10 2006 052 564 B3 und viele mehr.

Ein besonderes Problem bei der Mitralklappe und der Trikuspidalklappe ist die im Vergleich zur Aortenklappe ungleich komplexere und dynamischere Anatomie. Einerseits ist die sichere Befestigung der Klappenprothese viel schwieriger, weil im Bereich der Mitralklappe und der Trikuspidalklappe kein ausreichendes Widerlager besteht, wie es das zumeist stark verkalkte "Rohr" der Aorta darstellt. Vorschläge der Befestigung der Mitralklappenprothese an den vorhandenen Klappensegeln haben bei der praktischen Erprobung bereits zu Abrissen von Segeln oder Haltefäden und einer konsekutiven Embolisierung der Prothese geführt. Andererseits ragen die bisher praktisch erprobten Mitralklappenmodelle zu weit in die linke Herzkammer hinein, so dass die Abflussbahn der linken Herzkammer und die Aortenklappe behindert werden.

US 2007/0156233 A1 schlägt einen in zumindest einer Dimension an eine erkrnakte Herzklappe angepassten Stent zur Positionierung einer Klappenprothese vor. Die Befestigung erfolgt durch einen expandierbaren Ring, an dem die Klappen angebracht sind, zusätzlich werden in [0042], [0052] und [0053] "Dornen" oder Nähte vorgeschlagen. Laut EP 3 000 437 A1 fixiert ein Ballonförmiger Stent durch zumindest teilweisen Kontakt mit der Vorhofwandung eine Klappenprothese, wobei gemäß [0018] und [0020] die Klappe des Patienten nicht ersetzt sondern ergänzt werden soll. In US 2014/0330367 A1 wird ein an die Geometrie des Patienten individuell angepasster Stent zur Befestigung von Aortenklappenprothesen beschrieben, wobei der mehr oder weniger zylindrische Stent an der Aortenwandung anliegt und so die Aortenklappenprothese fixiert. Trotz der allgemeinen Behauptung in [0022] und [0077], man könne Mitral- und Trikuspidalklappen auf die gleiche Weise fixieren, lässt diese Schrift völlig offen, wie das realisiert werden könnte. Die US 2006/0271172 A1 beschreibt eine Klappenprothese, bei der zwei Ringe mit einem Stent verbunden werden, wobei der eine Ring oberhalb der andere Ring unterhalb der Klappe platziert wird. Die US 2014/0031928 A1 befasst sich mit einem Verschluss des Vorhofohres, und schlägt dazu ein "mesh" an einem Stent vor, wobei die Ostien nicht von dem Stent abgedeckt sein sollen. Des weiteren erstreckt sich der Stent durch die Klappe hindurch und stütztan sich gegenüberliegenden Enden beider Herzkammern ab. Diese Vorschläge sind für eine einfache und sichere sowie schonende Fixierung einer Mitral- oder Trikuspidalklapenprothese nicht ausreichend.

Es bestand daher weiter die Aufgabe, eine minimal-invasiv implantierbare Mitralklappenprothese oder Trikuspidalklappenprothese einfach und sicher zu befestigen.

Diese Aufgabe konnte erfindungsgemäß durch eine Klappenprothese gemäß Anspruch 1 gelöst werden, indem zur Positionierung und Fixierung der Klappenprothese ein selbstexpandierender, weitmaschiger Stent eingesetzt wird, welcher in seiner Form an die dreidimensional vermessene Geometrie des linken (für die Mitralklappe) bzw. rechten (für die Trikuspidalklappe) Vorhofs des Patienten angepasst wird und nach dem Implantieren an dessen Innenwand anliegt, wobei die Klappenprothese fest mit dem Stent verbunden ist.

Das besondere an diesem Stent ist, dass im Vergleich zu den bisherigen Vorschlägen nicht nur eine Anlage unmittelbar um den Mitralklappenbereich oder Trikuspidalklappenbereich herum gebildet wird, sondern der Stent im wesentlichen an der gesamten Wandung des Vorhofs anliegt, natürlich ausgenommen in dem Bereich, in dem die Klappenprothese angeordnet ist. Dadurch wird eine sehr sichere und zugleich schonende Befestigung erreicht.

Als Material für den Stent eignet sich jedes bekannte, biokompatible und selbstexpandierende Material, beispielsweise das im klinischen Einsatz langjährig bewährte Nitinol. Weiter muss der Stent für die Implantation zusammengedrückt in einen Katheter eingebracht werden können und nach dem Austritt aus dem Katheter die vorgesehene Form selbsttätig wieder einnehmen (sog. Memory-Effekt). Der Stent wird wie an sich bekannt von miteinander verbundenen, dünnen (zum Beispiel 0,1-0,2 mm dicken) Drähten gebildet. Es handelt sich erfindungsgemäß um besonders weitmaschige Stents, ansonsten sind der konkreten Anordnung und Form der Drähte keine Grenzen gesetzt.

Wichtig ist, dass der Stent gemäß der beim Patienten gemessenen dreidimensionalen Form des linken bzw. rechten Vorhofs gefertigt wird, d.h. anhand von dreidimensionalen Bildern des individuellen Patienten. Entscheidend ist die Form im entspannten Zustand, also die größten Abmessungen. Die Abmessungen des Stents werden dann jeweils über, insbesondere 5-10% über, den gemessenen Werten des Vorhofs gewählt, um einen dauerhaften leichten Druck nach außen zu gewährleisten. Es wäre zwar kommerziell interessant die Stents in der Form nur grob anzupassen bzw. eine geringe Anzahl unterschiedlich großer Stents bereitzuhalten, aber damit wäre die Positionierung nicht sicher genug bzw. es würde lokal zu viel Druck ausgeübt werden. Zudem handelt es sich in aller Regel um geplante Eingriffe mit ausreichender Zeit für die Vorbereitung.

Die Vermessung des Vorhofs zum Erhalt der dreidimensionalen Bilder kann mit an sich bekannten Verfahren erfolgen. Geeignet sind beispielsweise Computertomographie, Kernspintomographie sowie transthorakale und transösophagale Echokardiographie. Dabei sind die Vorhofkontraktionen in ihrem Ausmaß zu dokumentieren. Die endgültige Größe des Stents sollte dann in allen Richtungen 5-10% über der Größe des Vorhofs im entspannten Zustand liegen.

Die Klappenprothese ist fest mit dem Vorhofstent verbunden und wird so von diesem getragen und sicher positioniert. Die Art der Klappenprothese ist erfindungsgemäß nicht begrenzt. Alle bekannten Mitral- und Trikuspidalklappenprothesen sind geeignet, mit dem Vorhofstent gemäß der Erfindung verbunden zu werden. Bevorzugt wird eine Bioklappe, z.B. eine 3-Taschenklappe aus Rinderperikard, verwendet. Es ist von großem Vorteil, dass der größte Teil der Klappenprothese erfindungsgemäß im linken bzw. rechten Vorhof angeordnet ist und so weder die linksventrikuläre bzw. rechtsventrikuläre Ausflussbahn noch die Aortenklappe bzw. Pulmonalklappe oder deren jeweilige Halteapparate (Sehnenfäden) behindert werden.

Es ist bevorzugt die Klappenprothese ebenfalls selbstexpandierend auszubilden und besonders bevorzugt sie in ihrer Form ebenfalls individuell an den Patienten anzupassen. Das ist auf Basis der ohnehin benötigten dreidimensionalen Bilder leicht möglich. Anschließend wird der Durchmesser des Klappenringes z.B. 5-10% über dem gemessenen Durchmesser der lichten Weite an seiner späteren Position im Herzen gewählt.

Die Klappenprothese wird mit dem Stent in herkömmlicher Art fest verbunden und von diesem sicher in ihrer vorgesehen Position gehalten.

In einer Ausführungsform wird der dichte Abschluss der Klappenprothese zum Vorhof und zur Herzkammer von zwei Ringen gebildet. Die Ringe bestehen vorzugsweise aus PTFE. Der obere Ring wird im Vorhof positioniert und ist dicker als der in der Herzkammer liegende untere Ring.

In einer besonders vorteilhaften Ausführungsform umfasst der Vorhofstent einer Mitralklappenprothese für den linken Vorhof Patches, vorzugsweise aus PTFE, zum Verschluss der Vorhofohres und/oder eines Vorhofseptumdefektes. Das linke Vorhofohr ist ein häufiger Ausgangspunkt von Embolien, besonders bei Vorhofflimmern. Erfindungsgemäß lässt sich ein geeigneter Patch mit dem Vorhofstent verbinden und verschließt so das Vorhofohr sicher und dauerhaft. Ein z.B. bei der Vermessung festgestellter Vorhofseptumdefekt kann ebenfalls durch ein Patch am Stent sicher verschlossen werden. Beides wird durch die individuelle Anpassung des Stents und die dabei erstellten dreidimensionalen Bilder ermöglicht. Nur mit dem erfindungsgemäßen Vorgehen kann die Lage, Form und Größe des Vorhofohres und eines evtl. Septumdefektes genau genug bestimmt und der oder die Patches exakt positioniert und dicht genug angelegt werden. Mit allen bisherigen Vorschlägen war dies nicht möglich.

Zur Herstellung der erfindungsgemäßen Mitralklappenprothese werden zunächst dreidimensionale Bilder des linken Vorhof und der nativen Mitralklappe des Patienten angefertigt. Dann wird ein Vorhofstent gefertigt, der in seiner Form an die gemessene Form des Patientenvorhofes angepasst wird. Die Abmessungen des Stents werden so gewählt, dass sich nach seiner Implantation in den Vorhof ein gleichmäßiger, leichter Druck nach außen ergibt. Es wird eine geeignete Klappenprothese ausgewählt, die vorzugsweise ebenfalls auf Basis der dreidimensionalen Bilder individuell in Größe und Form angepasst ist, und mit dem Stent fest verbunden. Sofern nötig oder gewünscht kann auf Basis der dreidimensionalen Bilder ein Patch zum Verschluss des Vorhofohres an dem Stent angebracht werden. Ebenso kann ein Patch zum Verschluss eines Vorhofseptumdefektes an dem Stent angebracht werden.

Für eine Trikuspidalklappenprothese wird analog vorgegangen, wobei der Vorhofstent im rechten Vorhof angeordnet wird und dementsprechend dessen Abmessungen bestimmt werden und der Stent an diese angepasst wird. Auch bei einer Trikuspidalklappenprothese kann gewünschtenfalls ein Patch zum Verschluss eines Vorhofseptumdefektes am Stent angebracht werden.

Die Mitralklappen- bzw. Trikuspidalklappenprothese an dem Stent ist dann bereit zur Implantation mittels minimal-invasiver Techniken. Dazu wird sie in einen Katheter eingeführt. Mit Hilfe des Katheters erfolgt die Implantation z.B. transapikal entsprechend dem etablierten Vorgehen beim minimalinvasiven Ersatz der Aortenklappe oder transfemoral (über die Leistenvene.) Bei der Mitralklappe wird dabei die Punktion des Vorhofseptums erforderlich. Der dabei iatrogen entstehende Septumdefekt (dessen Durchmesser dem Einführkatheter entspricht) kann wie oben beschrieben durch einen zuvor geplanten PTFE-Patch mitverschlossen werden.

Die Erfindung soll anhand der beigefügten Figur erläutert werden, ohne jedoch auf die speziell beschriebene Ausführungsform beschränkt zu sein. Die Erfindung bezieht sich auch auf sämtliche Kombinationen von bevorzugten Ausgestaltungen, soweit diese sich nicht gegenseitig ausschließen. Die Angaben "etwa" oder "ca." in Verbindung mit einer Zahlenangabe bedeuten, dass zumindest um 10 % höhere oder niedrigere Werte oder um 5 % höhere oder niedrigere Werte und in jedem Fall um 1 % höhere oder niedrigere Werte eingeschlossen sind.

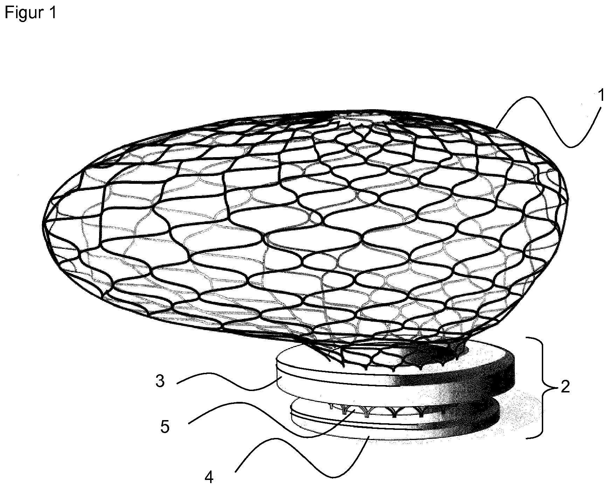

Figur 1 zeigt einen erfindungsgemäßen Stent 1 für den linken Vorhof mit einer fest verbundenen Mitralklappenprothese 2. Es ist deutlich zu sehen, dass der Stent 1 sehr weitmaschig ist, so dass er zugleich ausreichend fest anliegt und beim Herzschlag geringfügig zusammengedrückt werden kann, ohne die Herztätigkeit zu behindern. Der äußere Bereich der Klappenprothese 2 wird von zwei Ringen 3 und 4 gebildet, die mit einer zylindrischen Wandung 5 verbunden sind. Man erkennt gut, dass der Stent 1 in die Ringe 3 und 4 eingeformt und so sicher mit der Klappenprothese 2 verbunden ist. Im Inneren der Klappenprothese 2 befinden sich die eigentlichen Klappen, die in Figur 1 jedoch nicht erkennbar sind. Der größere Ring 3 dichtet die Klappenprothese 2 im Vorhof ab, der dünnere Ring 4 in der Herzkammer. Da sich bei der erfindungsgemäßen Prothese in der Herzkammer nur der dünne Ring 4 befindet, wird der Ausflusstrakt der Kammer nicht behindert.

## Patentansprüche

1. Minimal-invasiv implantierbare Mitralklappenprothese (2) oder Trikuspidalklappenprothese (2), mit einem selbstexpandierenden, weitmaschigen Stent (1), wobei die Klappenprothese (2) fest mit dem Stent (1) verbunden ist, **dadurch gekennzeichnet, dass** der Stent (1) in seiner Form an eine dreidimensional vermessene Geometrie eines linken oder rechten Vorhofs eines Patienten individuell angepasst ist und nach dem Implantieren an der Innenwand des Vorhofs des Patienten anliegt, wobei der Stent (1) die Klappenprothese (2) zur Positionierung und Fixierung trägt und die Abmessungen des Stents (1) über den gemessenen Werten gewählt werden, um einen dauerhaften leichten Druck nach außen zu gewährleisten.

2. Klappenprothese (2) gemäß Anspruch 1, **dadurch gekennzeichnet, dass** der Stent (1) aus einem biokompatiblen, selbstexpandierenden Material, vorzugsweise aus Nitinol, besteht.

3. Klappenprothese (2) gemäß Anspruch 1 oder 2, **dadurch gekennzeichnet, dass** die Abmessungen des Stents (1) 5-10% über den gemessenen Werten gewählt werden.

4. Klappenprothese (2) gemäß einem der Ansprüche 1 bis 3, **dadurch gekennzeichnet, dass** die Klappenprothese (2) eine Bioklappe ist.

5. Klappenprothese gemäß einem der Ansprüche 1 bis 3, **dadurch gekennzeichnet, dass** die Klappenprothese (2) eine 3-Taschenklappe aus Rinderperikard ist.

6. Klappenprothese (2) gemäß einem der Ansprüche 1 bis 5, **dadurch gekennzeichnet, dass** die Klappenprothese (2) selbstexpandierend ist.

7. Klappenprothese (2) gemäß einem der Ansprüche 1 bis 6, **dadurch gekennzeichnet, dass** die Klappenprothese (2) in ihrer Form individuell an den Patienten angepasst ist.

8. Klappenprothese (2) gemäß einem der Ansprüche 1 bis 7, **dadurch gekennzeichnet, dass** die Klappenprothese (2) von zwei mit einer zylindrischen Wandung verbundenen Ringen (3, 4) gebildet wird, wobei der Stent (1) in die Ringe (3, 4) eingeformt ist.

9. Klappenprothese (2) gemäß Anspruch 8, **dadurch gekennzeichnet, dass** die Klappensegel in dem Ring (3, 4) aus biokompatiblem Material, vorzugsweise aus Polytetrafluorethylen (PTFE), angeordnet sind.

10. Klappenprothese (2) gemäß einem der Ansprüche 1 bis 9, **dadurch gekennzeichnet, dass** der Vorhofstent (1) einen Patch, vorzugsweise aus PTFE, zum Verschluss des Vorhofohres umfasst.

11. Klappenprothese (2) gemäß einem der Ansprüche 1 bis 10, **dadurch gekennzeichnet, dass** der Vorhofstent (1) einen Patch, vorzugsweise aus PTFE, zum Verschluss eines Vorhofseptumdefektes umfasst.

12. Klappenprothese (2) gemäß einem der Ansprüche 1 bis 11, **dadurch gekennzeichnet, dass** es eine Mitralklappenprothese (2) ist, wobei der Stent (1) vorzugsweise einen Patch zum Verschluss des Vorhofohres und/oder eines Vorhofseptumdefektes aufweist.

13. Klappenprothese (2) gemäß einem der Ansprüche 1 bis 11, **dadurch gekennzeichnet, dass** es eine Trikuspidalklappenprothese (2) ist, wobei der Stent (1) vorzugsweise einen Patch zum Verschluss eines Vorhofseptumdefektes aufweist.

## Claims

1. A minimally invasive implantable mitral valve prosthesis (2) or tricuspid valve prosthesis (2) with a self-expanding, wide-meshed stent (1), wherein the valve prosthesis is firmly connected to the stent (1), **characterized in that** the stent (1) is individually adapted in its shape to a three-dimensionally measured geometry of a left or right atrium of a patient and, after implantation, lies against the inner wall of the atrium of the patient, wherein the stent (1) carries the valve prosthesis (2) for positioning and anchorage and the dimensions of the stent (1) are selected above the measured values in order to ensure a permanent slight outward pressure.

2. Valve prosthesis (2) according to claim 1, **characterized in that** the stent (1) consists of a biocompatible, self-expanding material, preferably of nitinol.

3. Valve prosthesis (2) according to claim 1 or 2, **characterized in that** the dimensions of the stent (1) are chosen 5 - 10 % above the measured values.

4. Valve prosthesis (2) according to any of claims 1 to 3, **characterized in that** the valve prosthesis (2) is a bio-valve.

5. Valve prosthesis according to one of claims 1 to 3, **characterized in that** the valve prosthesis (2) is a 3-pocket valve made of bovine pericardium.

6. Valve prosthesis (2) according to one of claims 1 to 5, **characterized in that** the valve prosthesis (2) is self-expanding.

7. Valve prosthesis (2) according to one of claims 1 to 6, **characterized in that** the valve prosthesis (2) is individually adapted in its shape to the patient.

8. Valve prosthesis (2) according to one of the claims 1 to 7, **characterized in that** the valve prosthesis (2) is formed by two rings (3, 4) connected with a cylindrical wall, wherein the stent (1) is molded into the rings (3, 4).

9. Valve prosthesis (2) according to claim 8, **characterized in that** the valve leaflets are arranged in the ring (3, 4) of biocompatible material, preferably of polytetrafluoroethylene (PTFE).

10. Valve prosthesis (2) according to any one of claims 1 to 9, **characterized in that** the atrial stent (1) comprises a patch, preferably of PTFE, for closing the atrial auricle.

11. Valve prosthesis (2) according to any one of claims 1 to 10, **characterized in that** the atrial stent (1) comprises a patch, preferably made of PTFE, for closing an atrial septal defect.

12. Valve prosthesis (2) according to any of claims 1 to 11, **characterized in that** it is a mitral valve prosthesis (2), wherein the stent (1) preferably comprises a patch for closing the atrial auricle and/or an atrial septal defect.

13. Valve prosthesis (2) according to any one of claims 1 to 11, **characterized in that** it is a tricuspid valve prosthesis (2), wherein the stent (1) preferably comprises a patch for closing an atrial septal defect.

## Revendications

1. Prothèse valvulaire mitrale (2) ou prothèse valvulaire tricuspide (2) implantable à invasion minimale, comportant un stent auto-expansible à larges mailles (1), dans lequel la prothèse valvulaire (2) est fermement reliée au stent (1), **caractérisé en ce que** la forme du stent (1) est individuellement adaptée en forme à une géométrie tridimensionnelle mesurée d'une oreillette gauche ou droite d'un patient et après l'implantation bute contre la paroi interne de l'oreillette du patient, dans laquelle le stent (1) porte la prothèse valvulaire (2) à des fins de positionnement et de fixation et les dimensions du stent (1) sont choisies au-dessus des valeurs mesurées afin d'assurer une légère pression permanente vers l'extérieur.

2. Prothèse valvulaire (2) selon la revendication 1, **caractérisée en ce que** le stent (1) est constitué d'un matériau biocompatible et auto-expansible, de préférence du nitinol.

3. Prothèse valvulaire (2) selon la revendication 1 ou 2, **caractérisée en ce que** les dimensions du stent (1) sont choisies supérieures de 5 à 10 % aux valeurs mesurées.

4. Prothèse valvulaire (2) selon l'une quelconque des revendications 1 à 3, **caractérisée en ce que** la prothèse valvulaire (2) est une valve biologique.

5. Prothèse valvulaire selon l'une quelconque des revendications 1 à 3, **caractérisée en ce que** la prothèse valvulaire (2) est constituée d'une valve à 3 poches produite à partir de péricarde bovin.

6. Prothèse valvulaire (2) selon l'une quelconque des revendications 1 à 5, **caractérisée en ce que** la prothèse valvulaire (2) est auto-expansible.

7. Prothèse valvulaire (2) selon l'une quelconque des revendications 1 à 6, **caractérisée en ce que** la prothèse valvulaire (2) est individuellement adaptée en forme au patient.

8. Prothèse valvulaire (2) selon l'une quelconque des revendications 1 à 7, **caractérisée en ce que** la prothèse valvulaire (2) est formée par deux anneaux (3, 4) reliés à une paroi cylindrique, dans laquelle le stent (1) est inséré dans les anneaux (3, 4).

9. Prothèse valvulaire (2) selon la revendication 8, **caractérisée en ce que** les feuillets valvulaires sont disposés dans l'anneau (3, 4) en matériau biocompatible, de préférence en polytétrafluoroéthylène (PTFE).

10. Prothèse valvulaire (2) selon l'une quelconque des revendications 1 à 9, **caractérisée en ce que** le stent auriculaire (1) comprend un patch, de préférence en PTFE, pour obturer l'appendice auriculaire.

11. Prothèse valvulaire (2) selon l'une quelconque des revendications 1 à 10, **caractérisée en ce que** le stent auriculaire (1) comprend un patch, de préférence en PTFE, pour obturer une communication interauriculaire.

12. Prothèse valvulaire (2) selon l'une quelconque des revendications 1 à 11, **caractérisée en ce qu'**il s'agit d'une prothèse valvulaire mitrale (2), dans laquelle le stent (1) comporte de préférence un patch pour obturer l'appendice auriculaire et/ou une communication interauriculaire.

13. Prothèse valvulaire (2) selon l'une quelconque des revendications 1 à 11, **caractérisée en ce qu'**il s'agit d'une prothèse valvulaire tricuspide (2), dans laquelle le stent (1) comporte de préférence un patch pour obturer une communication interauriculaire.
